**Europäisches Patentamt**

**European Patent Office**

**Office européen des brevets**

(11) Veröffentlichungsnummer: **0 174 456**

**A2**

(12) # EUROPÄISCHE PATENTANMELDUNG

(21) Anmeldenummer: 85108507.6

(22) Anmeldetag: 09.07.85

(51) Int. Cl.⁴: **A 61 B 5/10**
G 01 B 11/02, G 01 B 11/24

(30) Priorität: 13.07.84 DE 3425913

(43) Veröffentlichungstag der Anmeldung:
19.03.86 Patentblatt 86/12

(84) Benannte Vertragsstaaten:
AT BE CH FR GB IT LI NL SE

(71) Anmelder: Landwehr, Ulrich M.
Bahnhofstrasse 8
D-3000 Hannover 1(DE)

(72) Erfinder: Landwehr, Ulrich M.
Bahnhofstrasse 8
D-3000 Hannover 1(DE)

(74) Vertreter: Mende, Eberhard, Dipl.-Ing. et al,
Dipl.-Ing. Eberhard Mende Dipl.-Ing. Roger Döring
Patentassessoren Kabelkamp 20 Postfach 260
D-3000 Hannover 1(DE)

(54) Verfahren und Vorrichtung zur Ermittlung der Abmessungen eines Gegenstandes auf fotografischem Wege.

(57) Bei einem Verfahren zur Ermittlung der Abmessungen eines Gegenstandes auf photographischem Wege wird eine Photographie des Gegenstandes zusammen mit einem Maßraster hergestellt und während der Aufnahme ein schräg von oben einfallendes Muster aus horizintal verlaufenden Linien auf den Gegenstand projiziert. Das Maßraster wird dabei direkt auf die Bildebene bzw. den Film projiziert.

Fig 3

0174456

Ulrich M. Landwehr                          27. Juni 1984


Verfahren und Vorrichtung zur Ermittlung der Abmessungen
eines Gegenstandes auf photographischem Wege
_____


Die Erfindung betrifft ein Verfahren zur Ermittlung der Abmessungen eines Gegenstandes auf photographischem Wege, insbesondere der menschlichen Körpermaße, bei dem eine Photographie des Gegenstandes zusammen mit einem Maßraster hergestellt und gleichzeitig während der Aufnahme ein schräg von
oben einfallendes Muster aus horizontal verlaufenden Linien
auf den Gegenstand projiziert wird.

Aus der DE-OS 29 48 010 ist ein Verfahren gemäß dem Oberbegriff bekannt, bei dem auf der einen Hälfte der Photographie
der Gegenstand in der einen Stellung und auf der anderen
Hälfte der Photographie das Maßraster dargestellt wird, sodann
das Maßraster und der Gegenstand in ihren Positionen vertauscht werden, wobei der Gegenstand in einer anderen Stellung
angeordnet wird und in dieser Anordnung der ersten Aufnahme
überlagert wird. Diese Verfahrenstechnik hat sich als enorm
vorteilhaft gegenüber der bisherigen Vermessungstechnik erwiesen und ermöglicht eine genaue Vermessung. Insbesondere
in der Orthopädie läßt sich dieses Verfahren mit Vorteil einsetzen, da auf gesundheitsschädliches Röntgen verzichtet werden
kann. Mit dem genannten Verfahren lassen sich Haltungsschäden
bzw. Erkrankungen des menschlichen Knochengerüstes, wie Beckenschiefstand, Skoliose, ungleiche Beinlänge exakt feststellen
und vermessen und somit Heilerfolge objektiv durch Vergleichsmessungen beweisen. Der Nachteil dieses bekannten Verfahrens

- 2 -

ist darin zu sehen, daß durch die Überlagerung der beiden
Photoaufnahmen der Gegenstand bzw. die Person in ihrer Stellung verändert werden muß, was Menschen mit körperlichen Gebrechen schlecht zuzumuten ist.

Der vorliegenden Erfindung liegt von daher die Aufgabe zugrunde,
das bekannte Verfahren zu vereinfachen. Diese Aufgabe wird
dadurch gelöst, daß gemäß der Erfindung das Maßraster während
der Aufnahme direkt in die Bildebene projiziert wird. Im
Gegensatz zur bekannten Technik sind nunmehr keine zwei Aufnahmen, die sich einander überlagern, erforderlich, sondern
es reicht eine Aufnahme aus, die ein Maßraster zum Vermessen
des Gegenstandes aufweist. Wesentlich ist dabei, daß das Maßraster richtig einjustiert ist.

In Durchführung des erfindungsgemäßen Verfahrens hat es sich
als zweckmäßig erwiesen, das Maßraster mittels eines unmittelbar vor der Bildebene angeordneten Diapositivs auf die Bildebene zu projizieren. Das  Diapositiv besteht dabei aus einem
durchsichtigen Material wie Glas, in das zweckmäßigerweise
Metallfäden von wenigen Zehntelmillimetern Durchmesser eingegossen sind bzw. welches mit dem Raster bedruckt oder bemalt
ist. Auch ein Metallgitter unmittelbar vor der Bildebene erfüllt den gewünschten Zweck. Bei dieser Ausgestaltung erscheinen die Rasterlinien als schwarze Linien auf dem entwickelten
Film.

Alternativ kann es in Durchführung der Erfindung von Vorteil
sein, das Maßraster von der Seite mittels eines lichtdurchlässigen Spiegels durch das Objektiv auf die Bildebene zu
projizieren. Hierbei bedient man sich eines lichtdurchlässigen Spiegels, welcher zwischen dem Gegenstand und dem Objektiv
angeordnet ist. Der Spiegel ermöglicht die Aufnahme des
Gegenstandes, da er lichtdurchlässig ist und gleichzeitig
die Projektion des Maßrasters auf die Bildebene.

Die Erfindung betrifft weiterhin eine Vorrichtung zur Durchführung des Verfahrens. Die bekannte Vorrichtung besteht aus
einer Kamera und einem oberhalb der Kamera angebrachten, das
Linienmuster erzeugenden Blitzlichtprojektor. Gemäß der
Lehre der Erfindung ist in der Kamera zwischen Objektiv
und Bildebene ein das Maßraster aufweisendes Diapositiv in
einem Abstand von weniger als 0,5 mm, vorzugsweise weniger
als 0,2 mm parallel zur Bildebene angeordnet. Damit die
Linien des Maßrasters scharf in der Bildebene erscheinen,
sollte der Abstand zwischen dem Maßraster und der Bildebene
so gering wie möglich sein. Das Maßraster bzw. Diapositiv,
welches nahezu die gleiche Größe wie die Bildebene hat, besteht aus extrem dünnen Linien bzw. Metalldrähten. So sind
beispielsweise bis zu 60 horizizontal und bis zu 50 vertikal
verlaufende Rasterlinien auf dem Diapositiv angeordnet.

In einer anderen Ausgestaltung der erfindungsgemäßen Vorrichtung ist vorgesehen, zwischen dem Gegenstand und dem Objektiv
einen lichtdurchlässigen Spiegel unter einem Winkel von vorzugsweise 45$^o$ zur Bildebene und eine das Maßraster tragende
Platte senkrecht zur Bildebene und im Abstand zum Spiegel anzuordnen. Dabei ist es wesentlich, daß die Platte im Verlauf
des Strahlenganges den gleichen Abstand zum Objektiv aufweist
wie der Gegenstand. Anstelle der Platte kann aber auch vorteilhafterweise ein Diaprojektor mit einem das Maßraster aufweisenden Diapositiv  treten. Das Bild des Diapositivs, d.h.
das Maßraster erscheint auf der Bildebene scharf, wenn das
Objektiv des Diaprojektors richtig eingestellt ist.

Der wesentliche Vorteil der Erfindung ist darin zu sehen, daß
eine Doppelbelichtung nicht mehr erforderlich ist. Da das
Maßraster direkt in die Bildebene projiziert wird, ist eine
Veränderung der Stellung des Gegenstandes nicht notwendig.
Die Handhabung der erfindungsgemäßen Vorrichtung ist deshalb
gegenüber der bekannten Vorrichtung wesentlich einfacher.

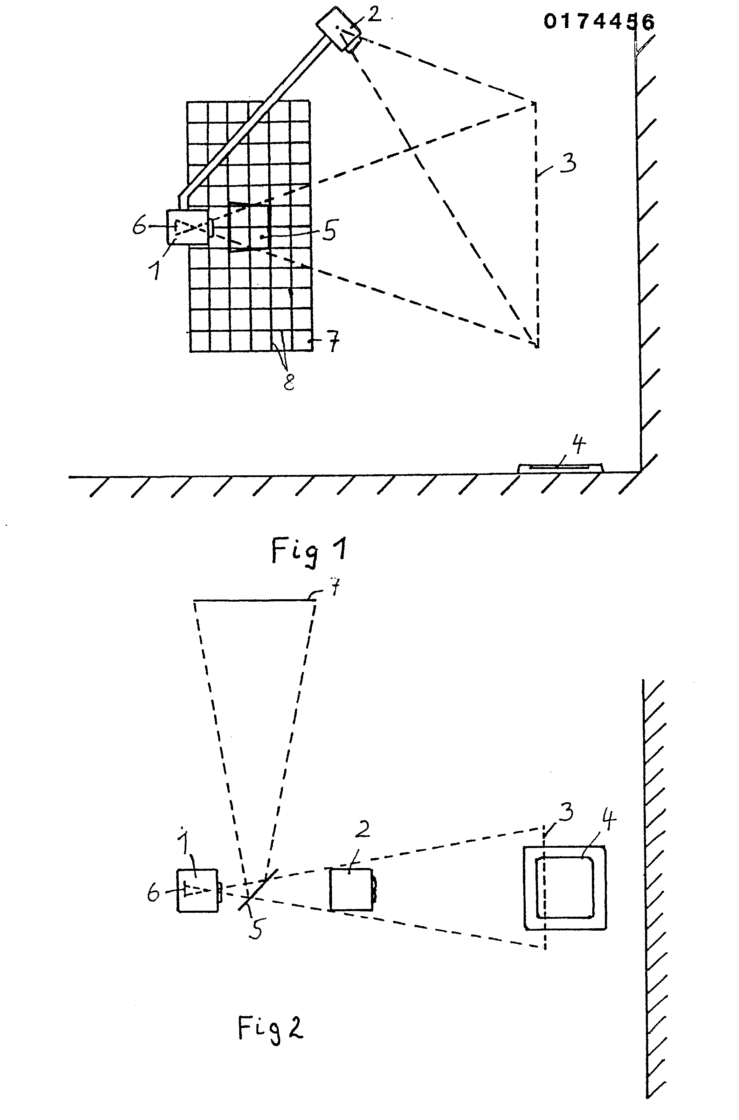

Die Erfindung ist anhand der in den Figuren 1 bis 4 schematisch dargestellten Ausführungsbeispiele näher erläutert.

In der Figur 1, die eine seitliche Ansicht einer Ausführungsform der Erfindung zeigt, ist mit 1 eine Kamera, beispielsweise eine Sofortbildkamera, bezeichnet. Oberhalb der Kamera 1 ist ein Blitzlichtprojektor 2 angeordnet, der wie in der DE-OS 29 48 010 näher beschrieben, ein Linienmuster während der Aufnahme auf den darzustellenden Gegenstand 3 projiziert. Mit 4 ist eine sogenannte Balancewaage bezeichnet, die bei der Vermessung eines Menschen anstelle des Gegenstandes 3 die Normalstellung des Menschen während der Aufnahme gewährleisten soll. Die Balancewaage ist in der DE-OS 33 01 864 näher beschrieben. Unmittelbar vor der Kamera 1 ist ein lichtdurchlässiger Spiegel 5 unter einem Winkel von 45$^\circ$ zur Ebene des Gegenstandes 3 bzw. zur Bildebene 6, mit dessen Hilfe ein auf einer Platte 7 dargestellte Maßraster 8 in die Kamera 1 während der Aufnahme eingespiegelt wird. Damit ein scharfes Bild des Maßrasters 8 in der Bildebene entsteht, ist es erforderlich, daß der Abstand des Gegenstandes 3 und der Abstand der Platte 7 zur Bildebene 6 in Richtung des Strahlenganges gesehen annähernd gleich ist.

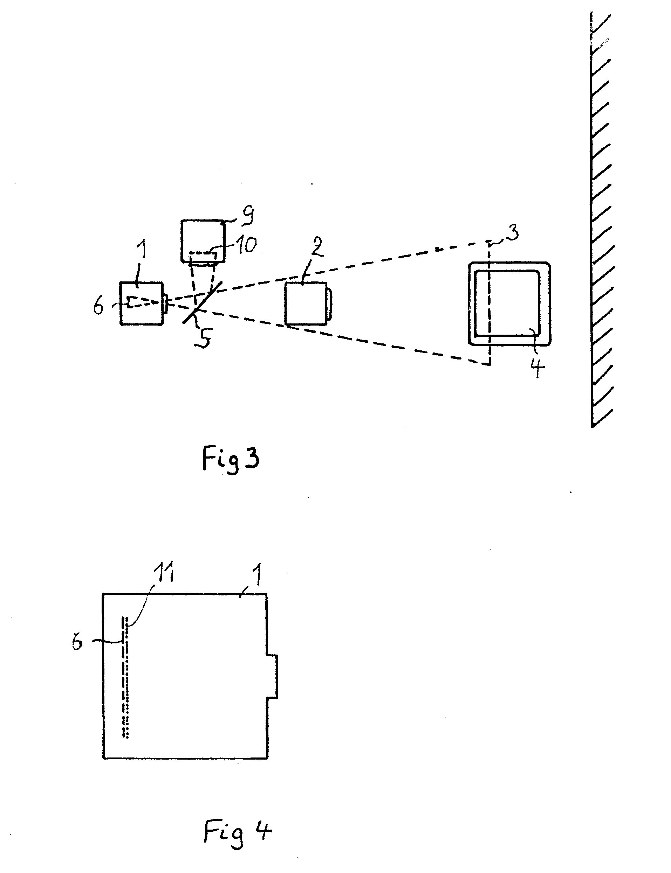

In Abänderung zu dieser Ausgestaltung ist in Figur 3 ein Diaprojektor 9 mit einem das Maßraster aufweisenden Diapositiv 10. Der Diaprojektor 9 projiziert das Bild des Maßrasters über den Spiegel 5 auf die Bildebene 6. Dies geschieht gleichzeitig mit der Aufnahme. Da der Spiegel 5 lichtdurchlässig bzw. halbdurchlässig ist, wird auch das Bild des Gegenstandes 3 auf der Bildebene 6 erzeugt. Das Maßraster 8 auf der Platte 7 kann sowohl hell auf dunklem Untergrund als auch umgekehrt ausgeführt sein. Auch das Diapositiv 10 kann z.B. eine geschwärzte Glasplatte sein, auf der die Maßrasterlinien eingeritzt sind. Umgekehrt könnte man auch eine klare Glasplatte verwenden, auf der die Maßrasterlinien aufgemalt, aufgedruckt oder in Form von extrem dünnen Drähten mit dem Glas verbunden sind.

In der Figur 4 ist ein Schnitt durch eine Kamera 1 dargestellt, die in einer Vorrichtung verwendet werden kann, die
ohne Spiegel 5 auskommt. Das Maßraster wird hier durch ein
unmittelbar vor der Bildebene 6 angeordnetes Diapositiv 11
erzeugt, welches aus einem durchsichtigen Werkstoff hergestellt ist, in den das Maßraster z.B. in Form von hauchdünnen Drähten eingearbeitet ist.

Damit die Rasterlinien,die auf dem Positivbild dunkel erscheinen, nicht verschwommen dargestellt werden, ist ein
Abstand von wenigen Zehntelmillimetern zur Bildebene 6 unumgänglich.

Ulrich M. Landwehr                    26.06.1984

## Patentansprüche

1. Verfahren zur Ermittlung der Abmessungen eines Gegenstandes auf photographischem Wege, insbesondere der menschlichen Körpermaße, bei dem eine Photographie des Gegenstandes zusammen mit einem Maßraster hergestellt und während der Aufnahme ein schräg von oben einfallendes Muster aus horizontal verlaufenden Linien auf den Gegenstand projiziert wird, dadurch gekennzeichnet, daß das Maßraster direkt auf die Bildebene bzw. den Film projiziert wird.

2. Verfahren nach Anspruch 1, dadurch gekennzeichnet, daß das Maßraster mittels eines unmittelbar vor der Bildebene angeordneten Diapositivs auf die Bildebene projiziert wird.

3. Verfahren nach Anspruch 1, dadurch gekennzeichnet, daß das Maßraster von der Seite mittels eines lichtdurchlässigen Spiegels durch das Objektiv auf die Bildebene projiziert wird.

4. Vorrichtung zur Durchführung des Verfahrens nach Anspruch 1 oder 2, bestehend aus einer Kamera und einem oberhalb der Kamera angebrachten, das Linienmuster erzeugenden Blitzlichtprojektor, dadurch gekennzeichnet, daß in der Kamera zwischen Objektiv und Bildebene ein das Maßraster aufweisendes Diapositiv in einem Abstand von weniger als 0,5 mm vorzugsweise weniger als 0,2 mm parallel zur Bildebene angeordnet ist.

5. Vorrichtung nach Anspruch 1 oder 3, bestehend aus einer Kamera und einem oberhalb der Kamera angeordneten das Linienmuster erzeugenden Blitzlichtprojektor, dadurch gekennzeichnet, daß zwischen dem Gegenstand und dem Objektiv ein licht-

- 2 -

durchlässiger Spiegel unter einem Winkel von vorzugsweise 45$^{\circ}$ zur Bildebene angeordnet ist und daß eine das Maßraster tragende Platte senkrecht zur Bildebene und im Abstand zum Spiegel angeordnet ist.

6. Vorrichtung nach Anspruch 5, <u>dadurch gekennzeichnet,</u> daß an die Stelle der Platte ein Diaprojektor mit einem das Maß-raster aufweisenden Diapositiv tritt.

0174456

Fig 1

Fig 2

Fig 3

Fig 4